# EUROPÄISCHE PATENTSCHRIFT

(11) **EP 3 862 754 B1**
(45) Veröffentlichungstag und Bekanntmachung des Hinweises auf die Patenterteilung: **02.04.2025**
(21) Anmeldenummer: 21156405.9
(22) Anmeldetag: 10.02.2021
(51) Int. Cl.: G01N 33/68

(54) **VERFAHREN ZUM NACHWEIS VON SCHLACHTNEBENPRODUKTEN UND/ODER SEPARATORENFLEISCH**
METHOD FOR DETECTING SLAUGHTER BYPRODUCTS AND/OR MECHANICALLY SEPARATED MEAT
PROCÉDÉ PERMETTANT DE DÉCELER LA PRÉSENCE D'ABATS ET/OU DE VIANDE DE SÉPARÉE MÉCANIQUEMENT

(30) Priorität: 10.02.2020 DE 102020103267
(43) Veröffentlichungstag der Anmeldung: 11.08.2021
(73) Patentinhaber: GfL Gesellschaft für Lebensmittel-Forschung mbH, 10787 Berlin (DE)
(72) Erfinder: Hofsommer, Mikko, 12101 Berlin (DE); Wittke, Stefan Johannes, 28357 Bremen (DE)
(74) Vertreter: Gulde & Partner

(56) Entgegenhaltungen:
- SUROWIEC I ET AL: "Proteomic approach for the detection of chicken mechanically recovered meat", MEAT SCIENCE, ELSEVIER SCIENCE, GB, vol. 89, no. 2, 6 April 2011 (2011-04-06), pages 233 - 237, XP028377824, ISSN: 0309-1740, [retrieved on 20110415], DOI: 10.1016/J.MEATSCI.2011.04.004
- GUO SHANGWEI ET AL: "A rapid and simple UPLC-MS/MS method using collagen marker peptides for identification of porcine gelatin", RSC ADVANCES, vol. 8, no. 7, 1 January 2018 (2018-01-01), pages 3768 - 3773, XP055805714, Retrieved from the Internet <URL:https://pubs.rsc.org/en/content/articlepdf/2018/ra/c7ra12539a> DOI: 10.1039/C7RA12539A
- DAY LI ET AL: "Detection of mechanically recovered chicken meat using capillary gel electrophoresis", MEAT SCIENCE., vol. 58, no. 1, 1 May 2001 (2001-05-01), GB, pages 31 - 37, XP055805759, ISSN: 0309-1740, DOI: 10.1016/S0309-1740(00)00127-3
- YUKI KUMAZAWA ET AL: "A novel LC-MS method using collagen marker peptides for species identification of glue applicable to samples with multiple animal origins", HERITAGE SCIENCE, BIOMED CENTRAL LTD, LONDON, UK, vol. 6, no. 1, 13 July 2018 (2018-07-13), pages 1 - 9, XP021258540, DOI: 10.1186/S40494-018-0209-Y
- Z. KHIARI ET AL: "Low molecular weight bioactive peptides derived from the enzymatic hydrolysis of collagen after isoelectric solubilization/precipitation process of turkey by-products", POULTRY SCIENCE, vol. 93, no. 9, 1 September 2014 (2014-09-01), Oxford, pages 2347 - 2362, XP055586362, ISSN: 0032-5791, DOI: 10.3382/ps.2014-03953
- TOMAIUOLO MICHELE ET AL: "Innovative techniques for identifying a mechanically separated meat: sample irradiation coupled to electronic spin resonance", EUROPEAN FOOD RESEARCH AND TECHNOLOGY, SPRINGER BERLIN HEIDELBERG, BERLIN/HEIDELBERG, vol. 245, no. 10, 1 August 2019 (2019-08-01), pages 2331 - 2341, XP036904794, ISSN: 1438-2377, [retrieved on 20190801], DOI: 10.1007/S00217-019-03340-X
- POSPIECH MATEJ ET AL: "An Innovative Detection of Mechanically Separated Meat in Meat Products", FOOD ANALYTICAL METHODS, SPRINGER NEW YORK LLC, US, vol. 12, no. 3, 10 November 2018 (2018-11-10), pages 652 - 657, XP036701909, ISSN: 1936-9751, [retrieved on 20181110], DOI: 10.1007/S12161-018-1394-8
- IAMMARINO MARCO ET AL: "Innovative approaches for identifying a mechanically separated meat: evaluation of radiostrontium levels and development of a new tool of investigation", JOURNAL OF FOOD SCIENCE AND TECHNOLOGY, SPRINGER (INDIA) PRIVATE LTD, INDIA, vol. 57, no. 2, 3 September 2019 (2019-09-03), pages 484 - 494, XP037019970, ISSN: 0022-1155, [retrieved on 20190903], DOI: 10.1007/S13197-019-04076-Y
- KOISTINEN KAISA M. ET AL: "Birch PR-10c is induced by factors causing oxidative stress but appears not to confer tolerance to these agents", NEW PHYTOLOGIST, vol. 155, no. 3, 20 August 2002 (2002-08-20), GB, pages 381 - 391, XP093078567, ISSN: 0028-646X, Retrieved from the Internet <URL:https://onlinelibrary.wiley.com/doi/full-xml/10.1046/j.1469-8137.2002.00481.x> DOI: 10.1046/j.1469-8137.2002.00481.x
- NALAZEK-RUDNICKA KATARZYNA ET AL: "MRM-MS of marker peptides and their abundance as a tool for authentication of meat species and meat cuts in single-cut meat products", FOOD CHEMISTRY, vol. 283, 8 January 2019 (2019-01-08), pages 367 - 374, XP085590279, ISSN: 0308-8146, DOI: 10.1016/J.FOODCHEM.2019.01.007

## Beschreibung

Die vorliegende Patentanmeldung betrifft ein Verfahren zum Nachweis von Schlachtnebenprodukten und/oder Separatorenfleisch in einer Probe gemäß dem Oberbegriff des Anspruchs 1.

In der Lebensmittelindustrie kommt es regelmäßig zur Substitution von hochpreisigen Inhaltsstoffen durch niedrigpreisige Inhaltsstoffen, zum Beispiel durch Fleisch einer anderen Spezies oder durch Schlachtnebenprodukte wie beispielsweise Separatorenfleisch. Die Bezeichnung Separatorenfleisch beschreibt Fleischteile, die maschinell vom Knochen gelöst wurden. Bedingt durch das maschinelle Verfahren enthält Separatorenfleisch neben Fleisch auch Bandscheibenmaterial sowie aber beispielsweise auch Knochen und Knochenhaut und/oder Knochenmark. Separatorenfleisch von Spezien wie Rindern, Ziegen und Schafen darf in vielen Ländern aufgrund medizinischer Bedenken nicht in der Lebensmittelproduktion verwendet werden. Bei Schlachtnebenprodukten kann es sich außerdem um andere Bestandteile von Tieren wie beispielsweise Bestandteile des Hirns, der Niere, der Leber, des Herzens, oder allg. von Innereien handeln.

Das Problem der Substitution hochpreisiger Inhaltsstoffe gewinnt an Bedeutung, da immer mehr Tierarten für die Lebensmittelproduktion verwendet werden. Dazu kommt, dass bei der Produktion von Lebensmitteln anfallende Reststoffe im Zuge der Abfallvermeidung zunehmend verwertet werden. Nach offiziellen Angaben hat Separatorenfleisch von Geflügel einen Marktanteil von nahezu 90% aus und von Schweinen von etwa 10%. Da der Verdacht besteht, dass nicht in allen Fällen die Deklarationspflichten für Separatorenfleisch erfüllt werden, besteht ein Bedarf an entsprechenden Nachweisverfahren.

Der Nachweis von Separatorenfleisch erfolgt derzeit in der Regel mikroskopisch über den visuellen Nachweis von Knochensplittern aus der Fleischprobe, die aufgrund der Herstellungsweise von Separatorenfleisch in den Fleischbrei gelangen können. Der Nachweis ist aufwendig und langwierig und weist nicht immer die wünschenswerte Zuverlässigkeit auf. Neben dieser Analysemethode kann auch ein erhöhter Calciumgehalt ein Indikator für Separatorenfleisch sein. Auch dieser Nachweis weist eine sehr begrenzte Sensitivität auf. Beide Nachweisverfahren erlauben keine quantitative Bestimmung des Anteils des zugesetzten Separatorenfleischs. Antikörperbasierte Verfahren haben sich in der Vergangenheit als ungeeignet erwiesen.

### Stand der Technik

1) SUROWIEC I ET AL: "Proteomic approach for the detection of chicken mechanically recovered meat", MEAT SCIENCE, ELSEVIER SCIENCE, GB, vol. 89, no. 2, 6 April 2011 (2011-04-06), pages 233 - 237, XP028377824, ISSN: 0309-1740 Offenbart wird ein Proteom-basiertes Verfahren zur Detektion von mechanisch separiertem Hühnchenfleisch.
2) GUO SHANGWEI ET AL: "A rapid and simple UPLC-MS/MS method using collagen marker peptides for identification of porcine gelatin", RSC ADVANCES, vol. 8, no. 7, 1 January 2018 (2018-01-01), pages 3768 - 3773, XP055805714 Offenbart wird ein Verfahren zum Nachweis Schweine-Gelatine mittels UPLC-MS/MS zur Sicherstellung von Halal-Vorschriften.
3) DAY LI ET AL: "Detection of mechanically recovered chicken meat using capillary gel electrophoresis", MEAT SCIENCE., vol. 58, no. 1, 1 May 2001 (2001-05-01), GB, pages 31 - 37, XP055805759, ISSN: 0309-1740
   Die Publikation beschreibt eine Studie zur Unterscheidung zwischen rohem, mechanisch gewonnenem Hühnerfleisch (MRM) und handentbeintem Hühnerbrustfleisch (HDM) mittels Kapillargel-Elektrophorese (CGE) insbesondere durch Nachweis des Hämoglobingehaltes
4) YUKI KUMAZAWA ET AL: "A novel LC-MS method using collagen marker peptides for species identification of glue applicable to samples with multiple animal origins", HERITAGE SCIENCE, BIOMED CENTRAL LTD, LONDON, UK, vol. 6, no. 1, 13 July 2018 (2018-07-13), pages 1 - 9, XP021258540
   Beschrieben wird eine Methode zur Untersuchung kollagenreicher Klebstoffe tierischer Herkunft, die beispielsweise in historischen Kunstwerken, insbesondere Gemälden, benutzt wurden.
5) KAISA H. KOISTINEN ET AL.: "Birch PR-10c is induced by factors causing oxidative stress but appears not to confer tolerance to these agents", New Phytologist (2002) 155: 381-391
   Beschrieben wird die Untersuchung von bekannten und neu gefundenen pathogenesebezogenen PR-10-Proteine aus Birken u.a. mittels zweidimensionaler Elektrophorese und Massenspektrometrie.
6) KATARZYNA NALAZEK-RUDNICKA ET AL.: MRM-MS of marker peptides and their abundance as a tool for authentication of meat species and meat cuts in single-cut meat products", Food Chemistry 283 (2019) 367-374
   Die Studie untersuchte die Häufigkeit von Proteinmarkern in verschiedenen Fleischstücken mittels multiple reaction monitoring mass spectrometry (MRM-MS) zur Authentifizierung von rohem und verarbeitetem Fleisch. Mittels der beschriebenen Untersuchungsmethoden wurden Peptide, die von Myoglobin (Mb) und Myosin (My) stammen, analysiert.

Der Erfindung liegt die Aufgabe zugrunde ein Verfahren zu schaffen, mit der ein zuverlässiger Nachweis von Schlachtnebenprodukten und/oder Separatorenfleisch in Fleischerzeugnissen möglich ist.

Diese Aufgabe wird durch ein Verfahren mit den Merkmalen des Anspruchs 1 gelöst. Die Erfindung betrifft demnach ein in vitro Verfahren zum Nachweis von Schlachtnebenprodukten und/oder Separatorenfleisch in einer Probe. Das erfindungsgemäße Verfahren weist im Wesentlichen die Schritte auf, wonach a) eine Probe bereitgestellt wird und b) mindestens ein Protein bzw. Proteinfragment (Peptid), das spezifisch für das Schlachtnebenprodukt und/oder das Separatorenfleisch ist, mittels eines geeigneten Messverfahrens nachgewiesen wird.

Vorzugsweise sieht es die Erfindung außerdem vor, dass in Vorbereitung für die Durchführung des Messverfahrens (Schritt b) ein oder mehrere in der Probe enthaltene Marker (Markerproteine oder Markerpeptide) mittels der Endoprotease Thermolysin in Peptidfragmente gespaltet werden, wobei mindestens eines der Peptidfragmente spezifisch für das Schlachtnebenprodukt und/oder das Separatorenfleisch ist.

Ein besonderes Ausführungsbeispiel der Erfindung kann vorsehen, dass als Messverfahren massenspektrometrische Verfahren, immunochemische Verfahren, wie insbesondere Western-Blots oder ELISA, oder ein Verfahren mittels Nachweis von natürlichen oder künstlich hergestellten Liganden verwendet werden.

Mittels des erfindungsgemäßen Verfahrens ist es möglich, Separatorenfleisch und andere unerwünschte Schlachtnebenprodukte wie z.B. Herz, Niere, Hirn, Leber oder andere Innereien usw. bereits in sehr kleinen Mengen nachzuweisen. Verstöße gegen lebensmittelrechtliche Vorschriften, wie z.B. Deklarationspflichten können so zuverlässig nachgewiesen werden. Im Gegensatz zu den derzeit üblichen Verfahren ermöglicht das erfindungsgemäße Verfahren den spezifischen und, sofern erforderlich, auch quantitativen Nachweis von Separatorenfleisch und anderen Schlachtnebenprodukten in Fleischprodukten. Dabei kann das erfindungsgemäße Verfahren beispielsweise auf üblichen LC-MS/MS-Geräten oder Geräten mit ähnlicher Sensitivität durchgeführt werden, sodass sich die Vorgehensweise zur routinemäßigen Überprüfung von Lebensmittel-(Fleisch- und Wurstwaren) und Futtermittelproben eignet.

Das erfindungsgemäße Verfahren ist ein *in vitro* Verfahren, dient also der Testung von tierischen Proben, insbesondere Fleischproben oder fleischhaltigen Proben, *ex vivo.*

Das Verfahren kann zum Nachweis von Separatorenfleisch und/oder eines anderen Schlachtnebenproduktes eingesetzt werden. Der Begriff "Schlachtnebenprodukte" umfasst alle tierischen Bestandteile eines geschlachteten Tiers mit Ausnahme des Zielfleisches. Das Zielfleisch ist in der Regel Muskelfleisch. Schlachtnebenprodukte umfassen somit u.a. Knochen, Bandscheiben als Knorpelmaterial, Haut, Herz, Niere, Gehirn, Blut und Teile und Mischungen derselben. Schlachtnebenprodukte können auch Fett und Fleisch geringerer Qualität umfassen, beispielsweise Fleisch aus einem anderen Gewebe als dem Zielfleisch. Sofern das Zielfleisch beispielsweise Brustmuskelfleisch ist, kann Schenkelmuskelfleisch ein Schlachtnebenprodukt sein. In der Regel wird allerdings Muskelfleisch im Rahmen der Erfindung als Zielfleisch angesehen. Separatorenfleisch ist ebenfalls ein Schlachtnebenprodukt. Separatorenfleisch ist ein Fleischprodukt, das Bandscheibenbestandteile, Knochenbestandteile, Knochenhaut und/oder Knochenmark umfassen kann. Vorzugsweise wird das erfindungsgemäße Verfahren zum Nachweis von Bandscheibenbestandteilen und/oder Hautbestandteilen eingesetzt.

Eine Ausführungsform der Erfindung kann es vorsehen, dass der Nachweis des Schlachtnebenprodukts und/oder des Separatorenfleischs in der Probe qualitativ und/oder quantitativ erfolgt. Mittels des erfindungsgemäßen Verfahrens ist es auch dann möglich, unerwünschte Bestandteile in einer Probe nachzuweisen, wenn das in der Probe enthaltene Separatorenfleisch bzw. das in der Probe enthaltene andere Schlachtnebenprodukt aus derselben Tierart stammt wie die Probe, insbesondere wie das in der Probe enthaltene Zielfleisch. So ist es beispielsweise mittels des erfindungsgemäßen Verfahrens möglich, zwischen Muskelfleisch und einem anderen Gewebe von derselben Tierart zu differenzieren.

Das andere Gewebe kann beispielsweise ausgewählt sein aus der Gruppe bestehend aus Knochen, Bandscheiben, Haut, Blut, Fett und Mischungen derselben. Vorzugsweise ist eine Differenzierung zwischen Muskelfleisch und Bandscheiben möglich.

Insbesondere ist es denkbar, dass die Probe und das Schlachtnebenprodukt bzw. das Separatorenfleisch von derselben Tierart, insbesondere von demselben Wirbeltier bzw. Warmblüter, vorzugsweise der gleichen Schweineart, Ziegenart, Rinderart, Schafsart, Vogelart, insbesondere der gleichen Hühnervogel- oder Putenvogelart, stammen.

In einer bevorzugten Ausführungsform ist die Tierart eine Geflügelvogelart. Das Verfahren ist in dieser Ausführungsform zum Nachweis von Geflügel-Separatorenfleisch und/oder eines anderen aus einer Geflügelart stammenden Schlachtnebenproduktes in einer Geflügelfleischprobe geeignet.

Das erfindungsgemäße Nachweisverfahren umfasst mehrere Schritte. In einem ersten Schritt wird eine Probe bereitgestellt, in der ein oder mehrere in der Probe enthaltene Marker, insbesondere Markerproteine oder Markerpeptide, nachgewiesen werden sollen. Der Nachweis der Markerproteine kann indirekt über den Nachweis von einem oder mehreren spezifischen Peptidfragmenten (Protein-spezifischen Peptidfragmente, proteospecific peptides) erfolgen, das oder die spezifisch für das jeweilige Markerprotein sind. Das Verfahren ist für sehr unterschiedliche Probenarten geeignet. So kann das Verfahren beispielsweise für hochreine Proteinproben verwendet werden oder auch für Fleischproben bzw. fleischhaltige Proben. Die Fleischproben bzw. fleischhaltigen Proben können direkt aus der Schlachtung von Tieren stammen oder bereits für den Vertrieb als Lebensmittel prozessiert sein. Die Probe kann beispielsweise ausgewählt sein aus der Gruppe bestehend aus Wurstwaren - wie Wurst - oder Fleischwaren - wie Hackfleisch.

In einem zweiten Schritt des Verfahrens können ein oder mehrere in der Probe enthaltene Markerproteine mittels einer oder mehrerer Endoproteasen in Peptidfragmente gespalten werden, wobei mindestens eines der Peptidfragmente spezifisch für das Separatorenfleisch und/oder das andere Schlachtnebenprodukt ist.

Der eine oder die mehreren Marker sind bevorzugt Proteine oder Peptide, die spezifisch für das nachzuweisende Separatorenfleisch bzw. das andere Schlachtnebenprodukt sind. Dies bedeutet, dass der oder die Marker in dem Separatorenfleisch oder anderen Schlachtnebenprodukt vorkommen, aber nicht oder nur zu einem unwesentlichen Anteil in anderen Bestandteilen der Probe, insbesondere in Muskelfleisch. Bevorzugt sind der oder die Marker spezifisch für ein Gewebe, das ausgewählt ist aus der Gruppe bestehend aus Bandscheiben, Muskelfleisch, Schenkel, Flügel, oder Haut eines der vorgenannten Tierarten. Der Marker kann beispielsweise ein Haut- oder Bandscheiben-spezifisches Protein oder Peptid sein, vorzugsweise ein Bandscheiben-spezifisches Protein oder Peptid.

In einer bevorzugten Ausführungsform ist das Markerprotein ein Kollagen. Im Rahmen der Erfindung wurde gefunden, dass sich die Kollagene besonders gut als Marker eignen, da sie je nach Herkunft (Haut, Bandscheiben, Knorpel) spezifische bzw. messbare Unterschiede aufweisen. Es ist in einer alternativen Ausführungsform auch denkbar, als Markerprotein Proteine auszuwählen, die durch unterschiedliche Post-translationale Modifikationen (PTM) in dem Separatorenfleisch bzw. dem anderen Schlachtnebenprodukt durch die an anderer Stelle hierin beschriebenen Endoproteasen in andere Peptidfragmente geschnitten werden, als dasselbe Protein mit anderer PTM in den anderen Bestandteilen der Probe.

Das eine oder die mehreren Markerproteine werden in einem Schritt mittels einer oder mehrerer Endoproteasen in Peptidfragmente gespalten. Es können beispielsweise eine, zwei, drei oder mehr Endoproteasen verwendet werden. Im Rahmen der Erfindung hat sich herausgestellt, dass die Verwendung einer einzigen Endoprotease, nämlich Thermolysin ausreichend ist. Es ist daher bevorzugt, dass die Spaltung in diesem Schritt nur Thermolysin als einzige Endoprotease verwendet.

Andere prinzipiell geeignete Endoproteasen sind Fachleuten bekannt und umfassen beispielsweise Lys-C, Glu-C, ASP-N, Chymotrypsin, Trypsin, und dergleichen. Diese und weitere geeignete Endoproteasen sind kommerziell erhältlich. Fachleute sind in der Lage, weitere geeignete Endoproteasen zu ermitteln. Erfindungsgemäß wird aus den nachstehend erläuterten Gründen die Endoprotease Thermolysin verwendet.

Es kann vorkommen, dass potentielle Endoprotease-Schnittstellen durch posttranslationale Modifikationen (PTM), wie beispielsweise Glykosylierungen, geschützt sind und die gewählte Endoprotease an der Spaltung gehindert wird. In diesen Fällen kann die betreffende Modifikation im Rahmen des erfindungsgemäßen Verfahrens vor der Spaltung in dem vorbeschriebenen Schritt von dem Markerprotein entfernt werden. Entsprechende Verfahren, zum Beispiel Deglykosylierungsverfahren, sind Fachleuten bekannt.

Mindestens eines der derart erzeugten Peptidfragmente ist spezifisch für das Separatorenfleisch und/oder das andere Schlachtnebenprodukt bzw. eines der oben genannten Tierbestandteile. Der Nachweis des Peptidfragments ist, mit anderen Worten, indikativ für das Vorliegen von Separatorenfleisch bzw. das andere Schlachtnebenprodukt, bevorzugt für Separatorenfleisch. Das Peptidfragment ist spezifisch für Bestandteile des Separatorenfleisches bzw. des anderen Schlachtnebenprodukts, die in der übrigen Probe nicht enthalten sind, beispielsweise für Bandscheibe, Haut, Blut, Knochen oder dergleichen (s.o.). Um dies sicherzustellen, kommt die Sequenz des Peptidfragments bevorzugt nur in den Proteinen des Separatorenfleischs bzw. des anderen Schlachtnebenprodukts vor, insbesondere nach Spaltung der Proteine mit der gewählten Endoprotease. Das Peptidfragment ist somit geeignet, Separatorenfleisch bzw. andere Schlachtnebenprodukte von übrigen Bestandteilen der Probe, beispielsweise Muskelfleisch, zu unterscheiden. Das erfindungsgemäße Verfahren kann insbesondere zwischen Muskelfleisch und Separatorenfleisch bzw. anderen Schlachtnebenprodukten unterscheiden.

In bevorzugten Ausführungsformen ist das Peptidfragment ein Peptid eines Kollagens, insbesondere ein Fragment von Collagen alpha-1(Il).

Es ist besonders bevorzugt, dass das spezifische Peptidfragment ausgewählt ist aus der Gruppe bestehend aus SEQ ID NOs von Kollagenen. Es versteht sich, dass auch Varianten dieser Peptidfragmente erfindungsgemäß verwendet werden können. Dazu wird ein MRM-Ansatz verwendet. D.h. aus dem Fragmentierungsmuster des Zielpeptides werden bspw. die 4-5, insbesondere 3 von 4 oder 3 von 5, bzw. eine Mehrzahl, intensivsten Signale ausgewählt und nur wenn diese Fragmentionen sämtlichst oder in der Mehrzahl nachweisbar sind, konnte das Molekül eindeutig identifiziert werden. Die weiteren Fragmentionen mit geringerer Signalintensität werden nicht betrachtet.

Um einen spezifischen Nachweis mittels Massenspektrometrie oder anderer Verfahren zu ermöglichen, weist das spezifische Peptidfragment bevorzugt eine Länge von 5 Aminosäuren oder mehr, vorzugsweise 7 Aminosäuren oder mehr, auf. Es ist darüber hinaus bevorzugt, dass das Peptidfragment eine Länge von 30 Aminosäuren oder weniger, bevorzugt 25 Aminosäuren oder weniger, stärker bevorzugt 20 Aminosäuren oder weniger, aufweist.

Die entstehenden Peptidfragmente können vor Schritt b) elektrophoretisch oder chromatographisch, vorzugsweise flüssigchromatographisch, aufgetrennt werden. Geeignete Trennverfahren sind Fachleuten bekannt und können beispielsweise eine flüssigchromatographische Trennung über eine LC-Säule umfassen. Bevorzugt werden in diesem Schritt Umkehrphasensäulen (wie C18-Säulen) verwendet. Zur chromatographischen Trennung wird bevorzugt ein Flüssigchromatographie (LC)-System verwendet, beispielsweise ein System das ausgewählt ist aus der Gruppe bestehend aus HPLC-, U(H)PLC-, micro-LC- und nano-LC-Systemen. Die Verwendung eines micro-LC-Systems, wie der Eksigent MicroLC 200 (AB Sciex), ist besonders bevorzugt. Alternativ kann zur Trennung auch die Kapillar(gel)elektrophorese verwendet werden.

Anschließend wird das spezifische Peptidfragment oder dessen Sequenz mittels Massenspektrometrie oder anderer Verfahren nachgewiesen. Es ist aber auch denkbar, dass ein Epitop des Peptids direkt im intakten Markerprotein (ohne Verdau) dem Nachweis dient. Selbstverständlich können in Rahmen des erfindungsgemäßen Verfahrens auch zwei oder mehr Peptidfragmente nachgewiesen werden, beispielsweise zwei, drei, vier oder mehr. Diese Peptidfragmente weisen jeweils die hierin beschriebenen Eigenschaften auf. So können z. B. zwei oder mehr Peptidfragmente nachgewiesen werden, die jeweils spezifisch das Separatorenfleisch und/oder das andere Schlachtnebenprodukt, insbesondere Bandscheiben, sind. Es ist aber auch denkbar, während des Verfahrens Peptidfragmente nachzuweisen, die spezifisch für unterschiedliche Gewebe sind, zum Beispiel jeweils ein Peptidfragment, das spezifisch für Haut, Bandscheibe und Muskelfleisch ist.

Geeignete massenspektrometrische Verfahren, die für den Nachweis der Peptidfragmente bzw. Markerproteine geeignet sind, sind Fachleuten bekannt. Die verwendeten Massenspektrometer können MALDI oder lonenspray (ESI) lonenquellen aufweisen. Als Detektionssysteme kommen sämtliche Massenspektrometriesysteme in Frage. Hierzu gehören z. B. lonenfallenmassenspektrometer (Quadrupol-Ionenfalle, Linear trap, Fouriertransformations-lonenzyklotronresonanz (FT-ICR), Orbitrap), Quadrupol-Massenspektrometer, Sektorfeldmassenspektrometer und Flugzeitmassenspektrometer. Diese Systeme ermöglichen den Nachweis der Marker über die Retentions- / Migrationszeit (durch die Kopplung an ein Chromatografie- bzw. Elektrophoresesystem) und die Molekülmasse (das m/z-Verhältnis).

Wie Fachleuten bekannt ist, wird dabei zur eindeutigen Identifikation der indikativen Marker bevorzugt durch Hintereinanderkoppeln von mehreren Massenanalysatoren oder durch Verwendung von lonenfallen eine Sequenzanalyse durchgeführt. Zahlreiche geeignete Kombinationen von Analysatoren sind derzeit für derartige Fragestellungen verfügbar. Das verwendete Massenspektrometriesystem kann beispielsweise ausgewählt sein aus der Gruppe bestehend aus Triple-Quadrupol- (QqQ; triple-quads), Tandem-Tandem-TOF- (TOF-TOF), TRAP-FTICR- und TRAP-Orbitrap-Systemen. Bevorzugt ist die Massenspektrometrie, insbesondere die Tandem-Massenspektrometrie oder die Verwendung eines qTOF-Spektrometers.

Besonders geeignet sind auch, im Rahmen dieser Erfindung verwendete, Hybridmassenspektrometer (triple-tof). In diesen wird durch ESI (Electrospray-Ionisation) ein Quasi-Molekülion produziert, welches im ersten Analysatorquadrupol isoliert und dann in der sogenannten Kollisionszelle oder Stoßkammer, die z. B. eine "linear ion trap" sein kann, angeregt wird. Durch Zusammenstöße (wie bspw. "collision induced dissociation") mit einem Inertgas (z. B. Stickstoff oder Argon) oder andere Fragmentierungstechniken wie z.B. ECD, ETD, FID usw. wird diesem Quasi-Molekülion Energie zugeführt, sodass dieses daraufhin sehr spezifisch zu anderen, leichteren Ionen zerfällt. Im dritten Analysator, der z. B. ein Flugzeitmassenanalysator sein kann, wird mindestens eines der Produkt-Ionen des im Quadrupol isolierten Quasi-Molekülions (engl. "parent ion") ermittelt. Durch den Nachweis mindestens eines der Fragmentionen können Rückschlüsse auf deren Struktur gezogen werden. Alternativ besteht zudem die Möglichkeit, selektiv nur ein oder mehrere bekannte(s) Fragmention(en) ("targeted approach") zu beobachten. Hierdurch kann sehr empfindlich und selektiv quantifiziert werden. Dieser als Multiple Reaction Monitoring (MRM) bezeichnete Ansatz ist erfindungsgemäß bevorzugt.

In der Regel wird für die massenspektrometrische Analyse die Probe in einem geeigneten Lösemittel aufgenommen. Dafür kann die Probe zunächst lyophilisiert werden. Die ggf. lyophilisierte Probe kann in einem Lösemittel, z.B. Wasser, gelöst und homogenisiert werden (z.B. Ultra Turrax, 15 000 rpm, 1 min). Anschließend kann die Probe durch Erhitzen denaturiert werden. Nach dem Abkühlen der Probe kann die Endoprotease hinzugegeben und mit der Probe inkubiert werden.

Alternativ kann das geeignete Lösungsmittel auch unmittelbar zu der Probe mit den Markerproteinen hinzugegeben werden und diese kann dann wie beschrieben weiter bearbeitet (Homogenisation, Erhitzen, Inkubation) werden. Es ist bevorzugt, dass die in der Probe enthaltenen Endoproteasen vor Beginn der massenspektrometrischen Analyse deaktiviert werden, zum Beispiel durch Änderung des pH-Wertes (z.B. Zugabe von Ameisensäure) oder mittels einer Denaturierung durch Erhitzen der Probe, zum Beispiel bei 100°C für 10 min. Geeignete Lösemittel sind unter anderem Wasser oder Acetonitril und Methanol. Wasser ist besonders bevorzugt. Fachleuten sind weitere geeignete Protokolle für die Probenvorbereitung bekannt.

Die Datenaufnahme kann zunächst im Full-Scan MS / MSMS-Modus (z. B. im IDA-Modus = intensity dependant acquisition) erfolgen. Sofern die Fragmente und insbesondere ihre Peptidmassen bereits bekannt sind, ist es wie zuvor beschrieben alternativ oder zusätzlich auch möglich, die Datenaufnahme mittels eines MRM-Ansatzes vorzunehmen.

Das für (Hühner-)Bandscheibe spezifische Peptidfragment kann eine Masse aufweisen, die ausgewählt ist aus der Gruppe u.a. bestehend aus 564.28 m/z (vorzugsweise 3fach geladen), 861.89 m/z (vorzugsweise 2fach geladen), 826.71 m/z (vorzugsweise 3fach geladen), 756.00 m/z (vorzugsweise 3fach geladen) und 559.94 m/z (vorzugsweise 3fach geladen). Vorzugsweise ist das für Bandscheibe spezifische Peptidfragment ein Fragment mit der Masse 546,28 m/z, bevorzugt dreifach geladen. Fachleuten ist bekannt, dass durch post-translationale Modifikationen unterschiedliche Peptidfragmente oder Proteolyseprodukte aus Proteinen mit derselben Primärsequenz entstehen können. Entsprechend sind auch von den obigen Angaben abweichende Peptidfragmente desselben Markerproteins von der Erfindung mit umfasst.

Ein für (Hühner-)Haut spezifisches Peptidfragment kann bspw. eine Masse aufweisen, die ausgewählt ist, aus einer Gruppe bestehend aus 650.98 m/z (vorzugsweise 3fach geladen), 484.50 m/z (vorzugsweise 4fach geladen), 518.01 m/z (vorzugsweise 4fach geladen) und 819.72 m/z (vorzugsweise 3fach geladen). Es sind jedoch auch andere Massen denkbar.

Ein für (Hühner-)Brustfleisch spezifische Peptidfragment kann bspw. eine Masse aufweisen, die ausgewählt ist, aus der Gruppe bestehend aus 575.25 m/z (vorzugsweise 2fach geladen), 427.90 m/z (vorzugsweise 3fach geladen), 588.63 m/z (vorzugsweise 3fach geladen) und 673.89 m/z (vorzugsweise 2fach geladen). Es sind jedoch auch andere Massen denkbar.

Der Nachweis kann erfindungsgemäß qualitativ und/oder quantitativ durchgeführt werden. So kann beispielsweise die Menge des jeweiligen Peptidfragments bestimmt werden, das Verhältnis zu einem anderen Peptid oder lediglich, ob das Peptidfragment in der Probe nachgewiesen werden konnte. Hierzu werden die experimentellen Rohdaten mit einem Algorithmus verarbeitet, der dazu bestimmt ist, ein gewünschtes Signal oder Signale zu extrahieren und grafisch als m/z-Verhältnisse darzustellen (Massenspektrum). Entsprechende Algorithmen sind in allen gängigen Software-Programmen für die Auswertung von massenspektrometrischen Analysen enthalten. Geeignet ist daher beispielsweise eine mit dem MS-System mitgelieferte Software, wie PeakView^{™} (Sciex).

Die Nachweisgrenze des verwendeten Massenspektrometers bezogen auf die absolut injizierte Menge (z. B. in einem Volumen von 5 µL) an Peptid liegt vorzugsweise bei 50 ng oder weniger, stärker bevorzugt 20 ng oder weniger, z. B. bei etwa 10 ng.

Der Nachweis des spezifischen Peptidfragments in Schritt (b) kann alternativ oder zusätzlich auch mittels anderer Verfahren erfolgen. Geeignete Verfahren umfassen beispielsweise immunochemische Verfahren, d.h. Verfahren, in denen zum Nachweis des spezifischen Peptidfragments mindestens ein Antikörper eingesetzt wird, der das spezifische Peptidfragment spezifisch bindet. Solche Antikörper können beispielsweise mittels einer Carrier-Hapten-Bindung generiert werden, in der das Peptidfragment an ein größeres Protein gebunden wird und anschließend mittels bekannter Verfahren ein Antikörper gegen diesen Komplex generiert wird. Der Antikörper kann ein monoklonaler oder polyklonaler Antikörper sein. Der Antikörper kann in Form eines Antiserums verwendet werden. Geeignete immunochemische Verfahren, die Antikörper verwenden, umfassen beispielsweise Western-Blots und ELISA. Weitere Verfahren sind Fachleuten bekannt. Es ist erfindungsgemäß bevorzugt, den Nachweis in Schritt (b) in Form eines Schnelltests, zum Beispiel auf einem Biochip, durchzuführen.

Geeignete weitere Verfahren für den Nachweis in Schritt (b) umfassen ferner einen Nachweis mittels natürlicher oder künstlich hergestellter Liganden, wie z. B. rekombinanter Antikörperfragmente, single-chain-Antikörper, single CDR Antikörper, oder mittels Rezeptoren.

Auf Basis dieses Nachweises kann anschließend eine Entscheidung über die Eigenschaften der Probe getroffen werden. Ist die Probe eine für den Vertrieb als Lebensmittel vorgesehene Fleisch- bzw. Wurstware, so kann mittels des Nachweises beispielsweise ermittelt werden, ob die Ware alle Deklarationspflichten erfüllt.

Es ist angedacht, zusätzlich zu dem oder den spezifischen Peptidfragmenten auch ein Kontrollpeptid in der Probe nachzuweisen. Das Kontrollpeptid im Sinne der Erfindung ist ein Peptid, das durch die beschriebene Spaltung sowohl aus Proteinen des Separatorenfleisches bzw. eines anderen Schlachtnebenproduktes als auch aus den übrigen Bestandteilen der Probe, vorzugsweise Muskelfleisch, erzeugt wird. Durch den Nachweis eines Kontrollpeptids könnte somit die Gesamtmenge eines Kontrollpeptids ermittelt werden. In einem weiteren Schritt kann beispielsweise die Menge des oder der spezifischen Peptidfragment(e) in Relation zu dem Kontrollpeptid gesetzt werden. Auf diese Weise könnte der Anteil des jeweiligen Markerproteins und damit des Separatorenfleisches bzw. des anderen Schlachtnebenproduktes an der Gesamtmenge der Probe ermittelt. Alternativ wäre es zur Quantifizierung möglich, die indikativen Markerpeptide zu synthetisieren und diese in unterschiedlichen Konzentrationen (ggf. als Spike in der Realprobe) zu vermessen um eine Kalibrationsreihe aufzustellen. Diese Kalibration könnte anschließend zur Quantifizierung des Anteils an Separatorenfleisch und/oder anderer Schlachtnebenprodukte eingesetzt werden.

In einem anderen Ausführungsbeispiel betrifft die Erfindung auch hierin beschriebene Peptidfragmente eines Markerproteins. Die Peptidfragmente umfassen jeweils einen Abschnitt der Sequenz eines Markerproteins. Vorzugsweise sind die Peptidfragmente, wie an anderer Stelle näher beschrieben, spezifisch für ein Markerprotein.

In den Figuren sind mögliche Ausführungsbeispiele der Erfindung dargestellt. Es zeigen:
Fig. 1
   eine Darstellung von Messergebnissen für ein Peptidfragment mit der Masse 756,02 m/z für den Nachweis von Bandscheibe im Probenmaterial 'reine Bandscheibe';
Fig. 2
   eine Darstellung von Messergebnissen für ein Peptidfragment mit der Masse 756,02 m/z für den Nachweis von Bandscheibe im Probenmaterial 'Geflügelfleischwurst'; und
Fig. 3
   eine Darstellung eines Vergleichs von Messergebnissen für ein Peptidfragment mit der Masse 756,02 m/z für einen Nachweis von Bandscheibe in einem Probenmaterial 'Geflügelfleischwurst' (A und B) und 'Haut' (C und D).

Weitere Vorteile, Kennzeichen und Merkmale der vorliegenden Erfindung werden bei der nachfolgenden detaillierten Beschreibung von Ausführungsbeispielen anhand der beigefügten Figuren deutlich. Allerdings ist die Erfindung nicht auf diese Ausführungsbeispiele beschränkt.

### Beispiel 1: Probenvorbereitung

In den folgenden Referenzsubstanzen wurden im Rahmen der vorliegenden Erfindung als Konzeptnachweis spezifische, d.h. indikative, Peptidfragmente nachgewiesen:
- Hühner-Brustfleisch
- Hühner-Haut
- Hühner-Bandscheiben
- Real-Hühnerwurstwarenprobe aus dem Supermarkt (Geflügelwurst).

Die Nachweisverfahren sind im Folgenden in diesem und den nachfolgenden Beispielen im Detail beschrieben.

Die oben genannten Proben wurden jeweils enzymatisch verdaut, um Fragmente zu erzeugen, die einer LC-MS/MS Analyse zugänglich waren.

### 1. Thermolysin-Verdau:

Der enzymatische Aufschluss von 0,3-0,5 g Probe mit Thermolysin erfolgte in Anlehnung an ein Protokoll von Yokohama et al ( Yokoyama et al.; Peptide Inhibitors for Angiotensin I-Converting Enzyme from Thermolysin Digest of Dried Bonito; Biosci. Biotech. Biochem., 56 (10), 1541-1545, Japan;1992 ). Die lyophilisierte Probe wurde in 8 ml ultrareinem Wasser (Labtower 30 EDI; Thermo Fisher Scientific, Dreieich, Deutschland) gelöst und homogenisiert (Ultra-Turrax, 15 000 U / min, 1 min). Anschließend wurden die Proben hitzedenaturiert (100°C, 10 min). Nach dem Abkühlen (40°C) wurden dann 3 mg Thermolysin (Geobacillus stearothermiphilus; Sigma-Aldrich, Hamburg, Deutschland) zugegeben und inkubiert (4h, 37°C). Schließlich wurde das Enzym durch Hitzedenaturierung (100°C, 10 min) deaktiviert. Der dadurch - nach Zentrifugation - entstehende Überstand wurde durch einen 0,45 µL-HPLC-Spritzenfilter filtriert.

### 2. Vorbereitung zur Messung:

Der Proteingehalt der Proben (Überstand) wurde bestimmt und die Proteinkonzentration aller Proben schließlich auf 2,0 mg / ml eingestellt und die Proben sind dann für die Untersuchung fertig vorbereitet.

### Beispiel 2: LC-MS/MS-Analyse

Es konnten für Hühner-Bandscheiben, Muskelfleisch und Hühnerhaut jeweils spezifische Peptidfragmente nachgewiesen werden.

Für die Analysen wurde ein MRM (multi reaction modus; targeted approach) - Ansatz entwickelt. Die Experimente wurden mittels eines micro-LC-MS/MS-Systems durchgeführt (TripleTOF^{®} 4600 System, AB Sciex). Das System umfasst ein Massenspektrometer mit positiver Elektrospray-Ionisation. Selbstverständlich können auch alternative Massenspektrometer, z.B. solche mit negativer ESI, verwendet werden. Die vorangehende chromatographische Trennung der Peptide wurde mit einer Umkehrphasensäule (reversedphase) durchgeführt, in der Regel C18. Die Verwendung einer Vorsäule, z.B. zur Abtrennung von Probenverunreinigungen, ist möglich, war aber nicht zwingend notwendig. Es stellte sich heraus, dass die Reinigung der Geräte nach Verwendung von Trypsin (nicht erfindungsgemäß) sehr aufwändig war. Die erfindungsgemäße Verwendung von Endoprotease Thermolysin war unter diesem Gesichtspunkt deutlich zu bevorzugen.

In einem ersten Schritt wurden Probe, wie bereits beschrieben, vorbereitet. Dabei wurde der Proteingehalt eingestellt. Bei dem Verfahren sollen sämtliche Peptide detektiert werden (LC.MS im IDA-Modus (FullScan)) und zwar für sämtliche Proben (Brust, Schenkel, Bandscheibe, Haut) der bspw. 20 Hühner. Dazu wurde bioinformatisch (Markerview (Sciex) vorgegangen, um die Daten der einzelnen Messungen zu kompilieren und nach Gewebetyp zu sortieren. Durch Vergleich der Gewebetypsgruppen wurden die gruppenspezifischen Markerpeptide gefunden und, wenn möglich, auf Basis der IDA-Daten (dann aber MSMS-Daten) identifiziert. Aus diesen Untersuchungen stammt die Liste mit den Markerpeptiden, die viel mehr als die 4 Beispielpeptide beinhaltet. Zur Entwicklung eines MRM für die ausgewählten besten vier Markerpeptide sind die Messbedingungen dann so zu optimieren, dass nur noch die Markerpeptide untersucht werden, sodass die Nachweisgrenze verbessert werden konnte. Die MSMS-Spektren, welche zur Identifikation der Marker notwendig sind, erhalten dadurch eine bessere Qualität.

Während der Analyse wurde der Autosampler gekühlt (4 °C), um Verluste der Analyten während der Messsequenz zu vermeiden. Die Probenschleife hatte ein Volumen von 5 µL und die Säulenofentemperatur betrug 35 °C. Mögliche Arbeitsparameter der LC sind in Tabelle 1 zusammengefasst.

**Tabelle 1: Parameter der LC und des Autosamplers (links) und Gradient der LC an rp18 mit LMA (Wasser/0.1% Ameisensäure) und LMB (Acetonitril/0.1% Ameisensäure) (rechts)**

| **Parameter LC / Autosampler** | | | **Gradient** | | |
|---|---|---|---|---|---|
| **Parameter** | **Wert** | | **Zeit [min]** | **LMA [%]** | **LMB [%]** |
| Vorlauf | 2 min | | 0 | 100 | 0 |
| Nachlauf | 1 min | | 3,5 | 97,5 | 2,5 |
| Proben schleife | 5 µl | | 12 | 73 | 27 |
| Injektionsvolumen | 8 µl | | 16 | 30 | 70 |
| Flussrate | 20 µl/min | | 22 | 5 | 95 |
| Säulenofentemperatur | 35 °C | | 27 | 5 | 95 |
| Autosamplertemperatur | 4 °C | | 28 | 100 | 0 |

Wie in Tabelle 1 gezeigt, wurde die HPLC mittels Gradientenchromatographie durchgeführt. Die Gradientenparameter sind ebenfalls in der Tabelle dargestellt. Für die Messung wurde eine Flussrate von 20 µl/min verwendet, Laufmittel A bestand aus Milli-Q-Wasser mit 0.1 % Ameisensäure und Laufmittel B aus Acetonitril mit 0.1 % Ameisensäure. Zur Steuerung wurde die Software Eksigent 4.2 (AB Sciex) verwendet.

Vor jeder Messung wurde das System equilibriert, indem die Probenschleife und das Injektionssystem mit drei Spülvorgängen gereinigt wurden. Der erste Spülvorgang erfolgte mit Methanol und 0.1 % Ameisensäure, die anderen beiden Spülvorgänge mit Milli-Q und 0.1 % Ameisensäure.

Anschließend konnten die getrennten Analyten mittels des Massenspektrometers nachgewiesen werden. Bei der ebenfalls verwendeten Triple-ToF-MS wurden die in Tabelle 2 dargestellten Einstellungsparameter für die Ionenquelle verwendet.

**Tabelle 2: ESI-Quellen-Parameter am Triple-Tof4600 (Beispiel)**

| **Parameter** | **Wert** |
|---|---|
| Spannung an der Ionenquelle | 5300 V |
| Temperatur in der Quelle | 450 °C |
| Curtaingasdruck | 40 kPa |
| Nebulizergas | 15 kPa |
| Heatinggas | 25 kPa |
| ESI-Modus | positiv |

Aufgenommen wurden die Daten zunächst im sogenannten Full-Scan im IDA-MS/MS-Modus. Tabelle 3 fasst die eingestellten MS und MS/MS-Parameter zusammen. Die interessanten Fragmente wurden anschließend mittels eines XIC (extracted ion chromatogramm, d.h. nur Daten für das Zielpeptid wurden dargestellt = targeted approach) analysiert und ein MRM-Modus wurde entsprechend programmiert (Tabelle 4).

**Tabelle 3: MS/MS-Parameter für das eingesetzte LC-MS-System im IDA-Modus**

| **Parameter** | **Wert** |
|---|---|
| Kollisionsgas | Stickstoff |
| Collision Energy | 10 |
| Collision Energy spread | 20 |
| Start Mass | 90 m/z |
| End mass | 1800 m/z |
| Declustering Potential | 1000 |

Die Datenanalyse erfolgte mittels bekannter bioinformatischer Verfahren und Programme (s.o.). Durch internen Abgleich der Full Scan-Messungen mittels der Software Markerview^{™} (Sciex) wurden Unterschiede zwischen verschiedenen Gewebetypen auf Peptide- / Proteinbasis identifiziert. Mit Hilfe dieser indikativen Peptide war nachfolgend der Nachweis z. B. von Bandscheibenmaterial (als Bestandteil von Separatorenfleisch) nicht nur in reinen Bandscheibenproben sondern auch in Realproben aus dem Supermarkt möglich.

### Beispiel 3: Nachweis spezifischer Peptide für Bandscheibe, Brustfleisch und Haut von Hühnern

Exemplarische Ergebnisse der LC-MS/MS-Messungen nach einem Verdau mit Thermolysin sind in Tabelle 4 zusammengefasst. Es werden jeweils nur 4 bis 5 beispielhafte Markerpeptide gezeigt, die sich bei der Differenzierung zwischen Brustfleisch und Haut bzw. Bandscheiben als besonders gut herausstellten, da sie spezifisch für eines der getesteten Gewebe waren und eine hohe Signalintensität zeigten. Spezifisch bedeutet in diesem Zusammenhang, dass sie in der betreffenden Probe ausschließlich oder in einem deutlich erhöhtem Maße vorhanden waren. Die Peptide waren in den jeweils anderen Geweben nur in einer mit dem Hintergrundrauschen vergleichbaren Menge nachweisbar.

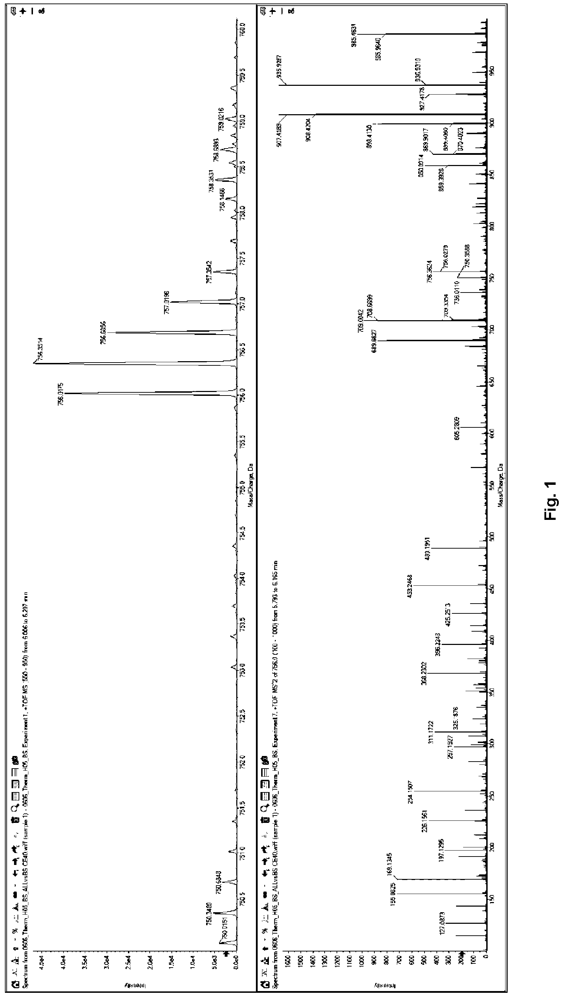

Beispielhaft sind in Fig. 1 Messergebnisse für das Peptidfragment mit der Masse 756,02 m/z für den Nachweis von Bandscheibe gezeigt. Gemessen wurde mit Thermolysin verdautes Probenmaterial, das aus reinem Bandscheibenmaterial bestand. Der obere Teil der Fig. 1 zeigt das MS-Spektrum der Messung. Daraus kann das m/z-Verhältnis des Peptidfragments - dem Marker - abgelesen werde, sowie dessen Ladung z. Da der Unterschied zwischen den Massen 0,33 ist, ist das Peptid 3-fach geladen. Der untere Teil der Fig. 1 zeigt das MSMS-Spektrum (Fragmentspektrum) für das Peptidfragment mit dem m/z-Verhältnis 756,02 und der Molekülmasse 2265 g/mol. Die in der untenstehenden Tabelle 4 aufgeführten spezifischen Peptidfragmente 689,66 / 708,66 / 907,41 / 935,92 (jeweils m/z) sind eindeutig zu erkennen. Da die Messmethode hier so eingestellt ist, dass ausschließlich das Mutterion (m/z 756,02) fragmentiert wird, stehen diese Massen in direktem Zusammenhang mit dem Marker und es ist zukünftig durch den Nachweis der Fragmentionen leicht möglich, das Mutterion eindeutig nachzuweisen (MRM-Messansatz). Die ausgewählten Peptidfragmente sind lediglich die vier Fragmentionen mit den höchsten Signalintensitäten und daher nicht die einzigen, die für das Mutterion (Peptidfragment) indikativ sind.

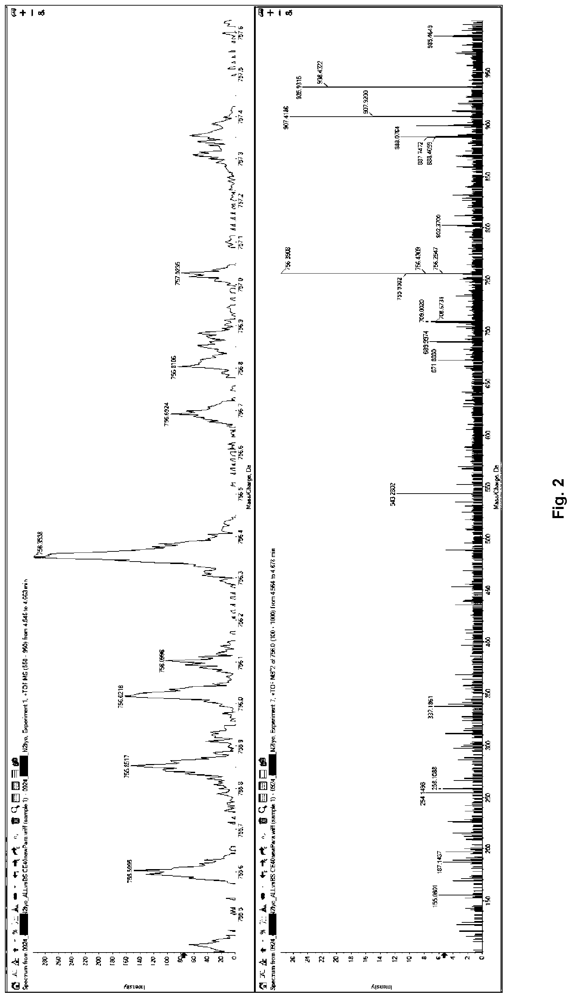

In Fig. 2 sind Messergebnisse für denselben Marker 756,02 m/z in einer Real-Hühnerwurstwarenprobe aus dem Supermarkt. In dem oberen Teil der Fig. 2 ist das MS-Spektrum gezeigt, in dem ein Peptid mit der grundsätzlichen Masse von 756,02 nachgewiesen werden kann. Zudem bedeutet der Unterschied von 0,33, dass es sich wie zuvor um ein 3-fach geladenes Molekül handeln muss. In der unteren Abbildung der Fig. 2 ist das MSMS-Spektrum (Fragmentspektrum) für den Marker mit dem m/z-Verhältnis Masse 756,02 und der Molekülmasse 2265 g/mol in der Hühnerfleischwurst gezeigt. Die in der Tabelle 1 aufgeführten Fragmente 689,66 (als erstes Isotopensignal bei 689,9974) / 708,66 / 907,41 / 935,92 sind eindeutig zu erkennen. Wie zuvor war die Messmethode hier so eingestellt, dass ausschließlich das Mutterion (m/z 756,02) fragmentiert wurde, daher stehen diese Massen direkt im Zusammenhang mit dem Marker und das Mutterion (der Marker) ist eindeutig nachgewiesen (MRM-Messansatz). Auch die weiteren, in der untenstehenden Tabelle gezeigten Marker für Bandscheibenmaterial waren auf diese Weise in der Probe eindeutig identifizierbar. Damit war anzunehmen, dass Bandscheibenmaterial in der Geflügelwurst vorhanden war. Aller Wahrscheinlichkeit nach bedeutete dies, dass die Wurst Hühnerseparatorenfleisch enthielt.

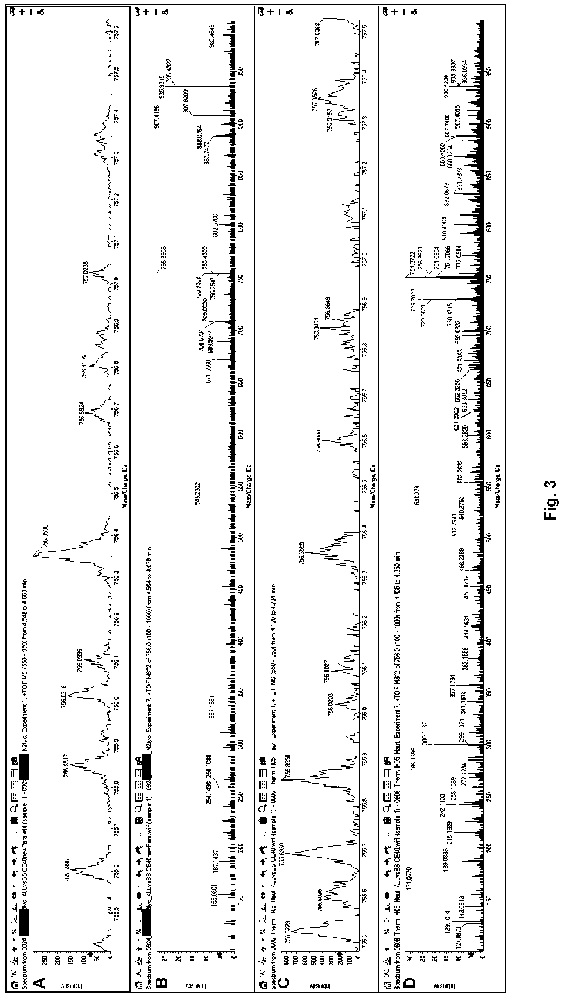

Diese Aussage wurde von den weiteren durchgeführten Analysen gestützt, da die ausgewählten Bandscheiben-Marker (vgl. Tabelle 1) ausschließlich in geringsten Spuren auch in Hautmaterial zu detektieren waren. Fig. 3 zeigt einen Vergleich von Messergebnissen nach dem bereits beschriebenen Schema in Hühnerfleischwurst ( Fig. 3 A und B) und in Hühnerhaut ( Fig. 3C und D ). Die Spuren des Markers in anderen Proben wie Haut lagen, wie in Fig. 3C und D erkennbar, stets im Bereich des Hintergrundrauschens, während sowohl in der Bandscheibenprobe als auch in der getesteten Geflügelfleischwurst ein deutlich erkennbares Signal für alle in der Tabelle gezeigten Bandscheibenmarker nachweisbar war ( Fig. 1, 2, 3A und B ). Die Kontamination der Geflügelfleischwurst kann daher nicht auf eine Kontamination mit Haut zurückgeführt werden.

In der Tabelle 4 sind drei Vergleiche dargestellt, wobei 'All vs BS' bedeutet, dass sämtliche Proben (Brustfleisch, Kratzfleisch, Haut) als eine Gruppe definiert wurden und von Bandscheibe als "Separatorenfleischmarker" abgegrenzt wurden. Die 5 angegebenen spezifischen Peptide sind mit anderen Worten indikative Marker für Bandscheibe.

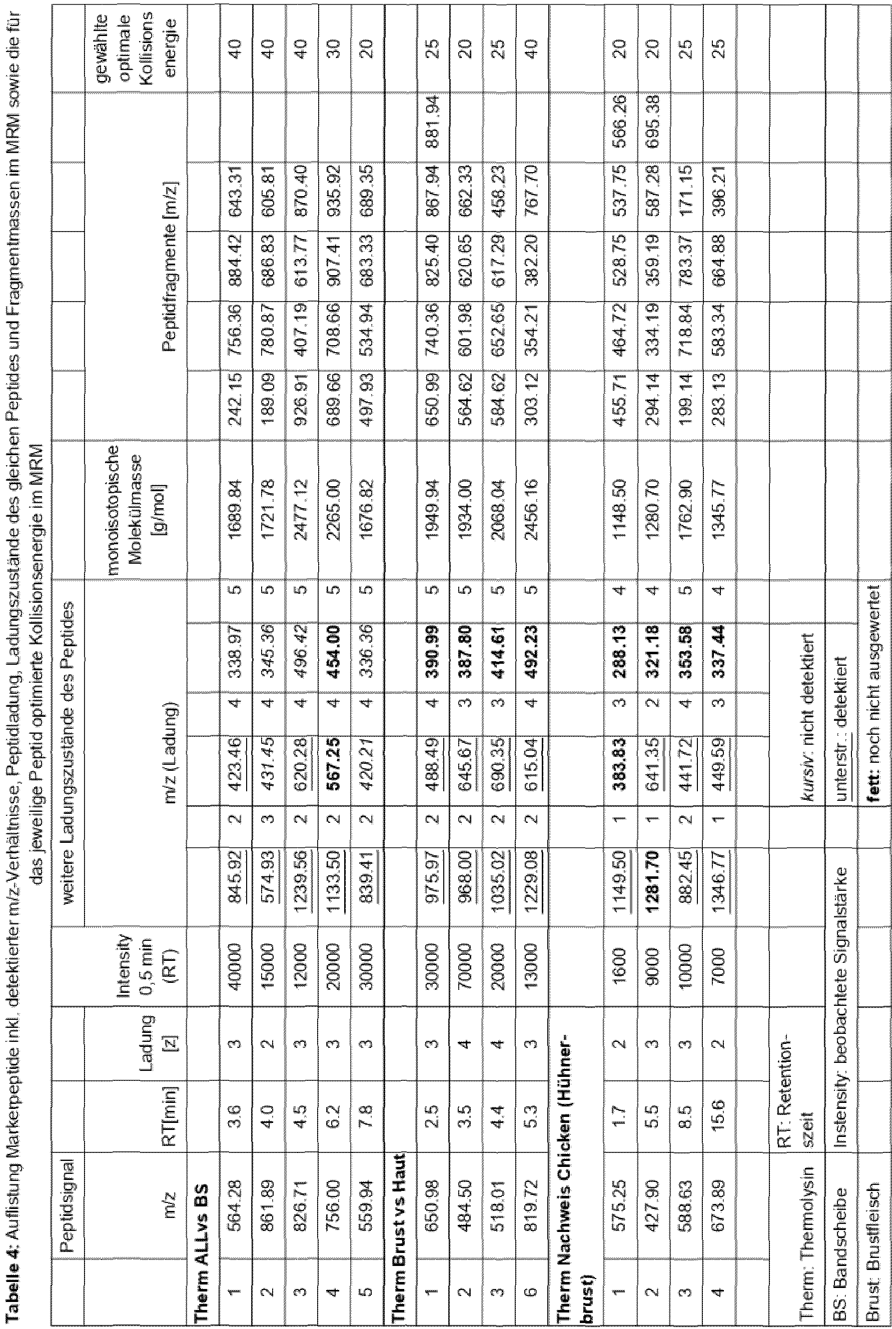

'Brust vs Haut' steht für Marker mit denen Brustfleisch von Hähnchenhaut abgegrenzt werden konnte. Da Haut ein Hauptbestandteil von Geflügelfleischwürsten ist, ist der Nachweis von Haut grundsätzlich interessant. 'Nachweis Chicken' bezeichnet die Marker, die für Brustfleisch spezifisch sind und bedeutet daher, dass Hühnchenbrust in der Probe vorhanden war. Die Retentionszeit (RT) sowie die Intensity (beobachtete Signalstärke) sind systemabhängig.

Anschließend wurden die Fragmentspektren mit Hilfe der ProteinPilot Software (ABSciex) ausgewertet und so die Proteinfragmente identifiziert. Dabei wurde festgestellt, dass es sich bei dem nachgewiesenen Proteinfragment 826,71 m/z (3fach geladen) um ein spezifisches Fragment des Markerproteins Collagen alpha-1(II) handelte. Das Fragment wurde dabei mit einer Confidence von 99% erkannt und dem Kollagen als Proteinspezifisches Peptid zugeordnet.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird. Die vorliegende Offenbarung schließt sämtliche Kombinationen der vorgestellten Einzelmerkmale ein.

## Patentansprüche

1. *In vitro* Verfahren zum Nachweis von Schlachtnebenprodukten und/oder Separatorenfleisch in einer Probe, **dadurch gekennzeichnet, dass** das Verfahren mindestens die Schritte umfasst, bei denen
a) eine Probe bereitgestellt wird; und
b) mindestens ein Protein bzw. Proteinfragment (Peptid), das spezifisch für das Schlachtnebenprodukt und/oder das Separatorenfleisch ist, mittels eines massenspektrometrischen Verfahrens nachgewiesen wird, wobei in Vorbereitung zur Durchführung des massenspektrometrischen Verfahrens (Schritt b) ein oder mehrere in der Probe enthaltene Markerproteine mittels Thermolysin in Proteinfragmente (Peptide) gespaltet werden, wobei mindestens eines der Peptide spezifisch für das Schlachtnebenprodukt und/oder das Separatorenfleisch ist.

2. Verfahren nach Anspruch 1, **dadurch gekennzeichnet, dass** die Probe und das Schlachtnebenprodukt bzw. das Separatorenfleisch von derselben Tierart, insbesondere von demselben Wirbeltier bzw. Warmblüter, vorzugsweise der gleichen Schweineart, Ziegenart, Rinderart, Schafsart, Vogelart, insbesondere der gleichen Hühnervogel- oder Putenvogelart, stammen.

3. Verfahren nach einem der vorangehenden Ansprüche, **dadurch gekennzeichnet, dass** der Marker ein Protein oder ein Peptid ist, das spezifisch für ein Gewebe ist, das ausgewählt ist aus einer Gruppe bestehend aus Muskelfleisch, Bandscheiben, Brust, Schenkel, Flügel oder Haut einer der genannten Tierarten.

4. Verfahren nach einem der vorangehenden Ansprüche, **dadurch gekennzeichnet, dass** der Marker ein Haut- oder Bandscheiben-spezifisches Protein bzw. Peptid ist, vorzugsweise ein Bandscheiben-spezifisches Protein oder Peptid einer der genannten Tierarten.

5. Verfahren nach einem der vorangehenden Ansprüche, **dadurch gekennzeichnet, dass** das Markerprotein ein Kollagen ist.

6. Verfahren nach einem der vorangegangenen Ansprüche, **dadurch gekennzeichnet, dass** der Nachweis des Schlachtnebenprodukts und/oder des Separatorenfleischs in der Probe quantitativ und/oder qualitativ ist.

7. Verwendung eines Analyseverfahrens zum Nachweis von Schlachtnebenprodukten und/oder Separatorenfleisch in einer Probe, mittels eines Verfahrens gemäß eines der vorangehenden Ansprüche.

## Claims

1. An in vitro method for detecting slaughter byproducts and/or mechanically separated meat in a sample, **characterized in that** the method comprises at least the steps in which
a) a sample is provided; and
b) at least one protein or protein fragment (peptide) which is specific to the slaughter byproduct and/or mechanically separated meat is detected by means of a mass spectrometry method, wherein, in preparation for performing the mass spectrometry method (step b), one or several marker proteins contained in the sample are split into protein fragments (peptides) by means of thermolysin, wherein at least one of the peptides is specific to the slaughter byproduct and/or mechanically separated meat.

2. The method according to Claim 1, **characterized in that** the sample and the slaughter byproduct or the mechanically separated meat stem from the same animal species, in particular from the same vertebrate or warm-blooded animal, preferably from the same pig species, goat species, bovine species, sheep species, bird species, in particular from the same chicken or turkey species.

3. The method according to any one of the preceding claims, **characterized in that** the marker is a protein or peptide specific to a tissue selected from a group consisting of muscle meat, intervertebral disk, breast, leg, wing or skin of one of the named animal species.

4. The method according to any one of the preceding claims, **characterized in that** the marker is a skin-specific or intervertebral disc-specific protein or peptide, preferably an intervertebral disc-specific protein or peptide of one of the named animal species.

5. The method according to any one of the preceding claims, **characterized in that** the marker protein is a collagen.

6. The method according to any one of the preceding claims, **characterized in that** the detection of the slaughter byproduct and/or mechanically separated meat in the sample is quantitative and/or qualitative.

7. A use of an analysis method for detecting slaughter byproducts and/or mechanically separated meat in a sample by means of a method according to any one of the preceding claims.

## Revendications

1. Procédé *in vitro* pour détecter des sous-produits d'abattage et/ou de la viande séparée mécaniquement dans un échantillon, **caractérisé en ce que** le procédé comprend au moins les étapes, dans lesquelles
a) un échantillon est fourni ; et
b) au moins une protéine ou un fragment de protéine (peptide), qui est spécifique au sous-produit d'abattage et/ou à la viande séparée mécaniquement est détecté au moyen d'un procédé de spectrométrie de masse ; en préparation à la mise en œuvre du procédé de spectrométrie de masse (étape b), une ou plusieurs protéines marqueurs contenues dans l'échantillon étant clivées en fragments de protéines (peptides) au moyen de thermolysine, au moins un des peptides étant spécifique du sous-produit d'abattage et/ou de la viande séparée mécaniquement.

2. Procédé selon la revendication 1, **caractérisé en ce que** l'échantillon et le sous-produit d'abattage ou la viande séparée mécaniquement proviennent de la même espèce animale, notamment du même vertébré ou animal à sang chaud, de préférence de la même espèce porcine, caprine, bovine, ovine, aviaire, notamment de la même espèce de poulet ou de dinde.

3. Procédé selon l'une quelconque des revendications précédentes, **caractérisé en ce que** le marqueur est une protéine ou un peptide spécifique d'un tissu choisi dans un groupe constitué par la viande musculaire, les disques intervertébraux, la poitrine, les cuisses, les ailes ou la peau de l'une desdites espèces animales.

4. Procédé selon l'une quelconque des revendications précédentes, **caractérisé en ce que** le marqueur est une protéine ou un peptide spécifique de la peau ou des disques intervertébraux, de préférence une protéine ou un peptide spécifique des disques intervertébraux de l'une desdites espèces animales.

5. Procédé selon l'une quelconque des revendications précédentes, **caractérisé en ce que** la protéine marqueur est un collagène.

6. Procédé selon l'une quelconque des revendications précédentes, **caractérisé en ce que** la détection du sous-produit d'abattage et/ou de la viande séparée mécaniquement dans l'échantillon est quantitative et/ou qualitative.

7. Utilisation d'un procédé d'analyse pour la détection de sous-produits d'abattage et/ou de viande séparée mécaniquement dans un échantillon, au moyen d'un procédé selon l'une quelconque des revendications précédentes.
